# EUROPEAN PATENT APPLICATION

(11) **EP 3 922 271 A1**
(43) Date of publication of application: **15.12.2021**
(21) Application number: 19921364.6
(22) Date of filing: 17.12.2019
(51) Int. Cl.: A61K 49/18

(54) **ULTRAFINE IRON OXIDE NANOPARTICLE-BASED MAGNETIC RESONANCE IMAGING T1 CONTRAST AGENT**

(30) Priority: 25.03.2019 KR 20190033476
(71) Applicant: Therabest Co., Ltd., Seoul 06656 (KR)
(72) Inventor: LEE, Yun Sang, Yongin-si Gyeonggi-do 16851 (KR); HYEON, Taeg Hwan, Seoul 06277 (KR); JEONG, Jae Min, Seoul 04425 (KR); LEE, Jae Sung, Seoul 06291 (KR); PARK, Ji Yong, Incheon 22002 (KR); KIM, Kyu Wan, Seoul 03663 (KR); SHIN, Jae Hwan, Yongin-si Gyeonggi-do 17000 (KR); KO, Guen Bae, Seoul 03123 (KR); LEE, Whal, Seoul 06505 (KR); PARK, Eun Ah, Seoul 06213 (KR)
(74) Representative: Prüfer & Partner mbB Patentanwälte · Rechtsanwälte
(86) International application number: PCT/KR2019/017870
(87) International publication number: WO 2020/197046

(57) **Abstract**

Provided is a T1 contrast agent for magnetic resonance imaging. The T1 contrast agent includes fine iron oxide nanoparticle cores and micelles encapsulating the core particles. The micelles include a nonionic surfactant consisting of a hydrophilic moiety containing at least two chains and a hydrophobic moiety containing at least one C₁₀-C₃₀ hydrocarbon chain. The T1 contrast agent of the present invention is a novel one based on fine iron oxide nanoparticles that can replace conventional gadolinium-based T1 contrast agents. The T1 contrast agent based on fine iron oxide nanoparticles according to the present invention is harmless to humans, is rapidly distributed in the blood, and has a uniform size, ensuring its uniform contrast effect. In addition, the T1 contrast agent of the present invention enables image observation for at least 1 hour to up to 2 hours and is excreted through the kidneys and liver. Therefore, the T1 contrast agent of the present invention avoids the problems encountered in conventional gadolinium-based contrast agents.

## Description

### Technical Field

The present invention relates to a T1 contrast agent for magnetic resonance imaging (MRI) based on ultrafine iron oxide nanoparticles, and more specifically to a T1 contrast agent for magnetic resonance imaging based on uniformly sized iron oxide nanoparticles whose surface is hydrophilized.

### Background Art

Magnetic resonance imaging (MRI) is a method for acquiring anatomical, physiological, and biochemical information on the body as images using a phenomenon in which the spins of hydrogen atoms are relaxed in a magnetic field. MRI is one of the current noninvasive diagnostic tools for real-time imaging of the body organs of living humans and animals.

For its diverse and precise use in the bioscience and medical fields, MRI is performed by introducing foreign materials into the body to increase the contrast of images. These materials are called contrast agents. Superparamagnetic and paramagnetic materials are used as contrast agents to contrast signals from body parts to be imaged by MRI so that the body parts can be clearly distinguished from their surroundings. Contrast between tissues on an MRI image arises because of different relaxations in the tissues. The relaxation is a phenomenon in which the nuclear spins of water molecules in the tissues return to their equilibrium state. A contrast agent affects the relaxations to create large differences in the degree of relaxation between the tissues and induces changes of MRI signals to make the contrast between the tissues clearer.

Enhanced contrast using contrast agents raises or lowers the intensities of image signals from specific living organs and tissues relative to their surroundings to provide clearer imaging of the organs and tissues. Positive contrast agents (or T1 contrast agents) refer to contrast agents that raise the intensities of image signals from body parts to be imaged by MRI relative to their surroundings. Negative contrast agents (or T2 contrast agents) refer to contrast agents that lower the intensities of image signals from body parts to be imaged by MRI relative to their surroundings. More specifically, MRI contrast agents are divided into T1 contrast agents using high spins of paramagnetic materials and T2 contrast agents using magnetic inhomogeneity around ferromagnetic or superparamagnetic materials.

T1 contrast agents are associated with longitudinal relaxation. Longitudinal relaxation is a phenomenon in which magnetization component Mz in the Z-axis direction of a spin absorbs an RF energy impact applied from the X-axis, is aligned with the Y-axis on the X-Y plane, and returns to its original value while releasing the energy to the outside. This phenomenon is also expressed as "T1 relaxation". The time for Mz to return to 63% of its initial value is referred to as a "T1 relaxation time". The shorter the T1 relaxation, the greater the MRI signal, indicating a shorter image acquisition time.

T2 contrast agents are associated with transverse relaxation. Magnetization component Mz in the Z-axis direction of a spin absorbs an RF energy impact applied from the X-axis, is aligned with the Y-axis on the X-Y plane, and returns to its original value while decaying the energy or releasing the energy to the surrounding spins. At this time, component My of the uniformly widened spin on the X-Y plane decays as an exponential function. This phenomenon is expressed as "T2 relaxation". The time for My to return to 37% of its initial value is referred to as a "T2 relaxation time". My decreases as a function of time and is measured by a receiver coil mounted on the Y-axis. The measured value is called a "free induction decay (FID) signal". A tissue with a short T2 relaxation time appears darker on the MRI.

Currently commercially available positive and negative MRI contrast agents use paramagnetic compounds and superparamagnetic nanoparticles, respectively. Iron oxide nanoparticles such as superparamagnetic iron oxide (SPIO) nanoparticles are currently used for T2 contrast agents. T2 contrast is a negative contrast that makes a target area darker than its surroundings. The contrast effect of T2 contrast is not much significant. Another disadvantage of T2 contrast is that a larger area than the actual area is contrasted due to the blooming effect. Meanwhile, T1 contrast agents are based on positive contrast and have an advantage in that images of desired sites appear bright. High spin materials, usually gadolinium complexes with 7 hole spins in the 4f orbital, are used for T1 contrast agents.

Gadolinium (Gd)-based contrast agents have already been developed in the 1970s. Since then, however, there have been no significant technological advances in the development of contrast agents. Recently, gadolinium-based contrast agents have been reported to cause side effects such as irreversible skin and organ sclerosis due to the toxicity of free gadolinium. Another recent report revealed that gadolinium was permanently deposited in brain tissues of patients who had been injected with an MRI contrast agent. Under these circumstances, the risk of gadolinium contrast agents has emerged. Although the prevalence of vascular diseases in patients with chronic renal failure and their mortality are high, the use of Gd-contrast agents is prohibited due to their risk of causing nephrogenic systemic fibrosis. Thus, there is an urgent need to develop MRI contrast agents that can be safely used in patients with chronic renal failure.

Iron oxides used in superparamagnetic materials can be classified into two types according to their particle size: superparamagnetic iron oxides (SPIOs) having a particle size of 50 nm or more and ultrasmall superparamagnetic iron oxides (USPIOs) having a particle size smaller than SPIOs. Smaller USPIO tends to stay in the blood vessels for a longer time because it is less susceptible to the phagocytic action of macrophages in the blood vessels. Based on this tendency, USPIO can be used to determine whether the blood vessels are normal or not. USPIO can be injected in a small amount compared to SPIO, allowing its rapid injection. USPIO reduces T1 and T2 to a similar degree, resulting in an increase in the signal intensity in T1-weighted images and a decrease in the signal intensity in T2-weighted images. Many contrast agents such as Feridex have been clinically used. Most of these contrast agents are synthesized by co-precipitation methods and suffer from limitations such as poor crystallinity, leading to inferior magnetic properties and non-uniform size.

Since the late 1990s, pyrolysis methods have newly developed to synthesize iron oxide nanoparticles with uniform sizes of 5 to 20 nm. Despite the reported fact that iron oxide nanoparticles synthesized by pyrolysis methods have better MRI T2 contrast effects than nanoparticles developed by co-precipitation methods, T1-weighted images are preferentially used in clinical applications compared to T2-weighted images because T1-weighted images are more accurate and T2-weighted images suffer from severe signal interference. For these reasons, there is a need to develop nanoparticles whose T1 contrast effect is comparable to or better than that of gadolinium-based contrast agents and which can replace gadolinium-based contrast agents.

### Detailed Description of the Invention

### Means for Solving the Problems

One object of the present invention is to provide ultrafine iron oxide nanoparticles that are prepared based on a combination of a technique for uniform core synthesis and a technique for surface hydrophilization and have a uniform contrast effect while maintaining their uniform size *in vivo,* thus being suitable for use in the production of T1 contrast agents that have the potential to replace conventional gadolinium-based contrast agents.

A further object of the present invention is to provide a platform for multiplex assays using ultrafine iron oxide nanoparticles.

Another object of the present invention is to provide a method for producing nanoparticles for multiplex assays whose surface is modified with a hydrophilizing material and a functionalizing material.

According to one aspect of the present invention, there is provided a T1 contrast agent for magnetic resonance imaging including fine iron oxide nanoparticle cores and micelles encapsulating the core particles wherein the micelles include a nonionic surfactant consisting of a hydrophilic moiety containing at least two chains and a hydrophobic moiety containing at least one hydrocarbon chain.

In one embodiment of the present invention, the cores may have a diameter of 6 nm or less and a polydispersity index (PDI) of 0.2 or less.

In a further embodiment of the present invention, the hydrophilic moiety may contain a polyalkylene glycol chain.

In another embodiment of the present invention, the nonionic surfactant may be a polysorbate surfactant.

In another embodiment of the present invention, the contrast agent may have an overall diameter of 10 nm or less and a PDI of 0.2 or less.

The present invention provides a platform for multiplex assays with a magnetic resonance imaging T1 contrast effect, including fine iron oxide nanoparticle cores and micelles encapsulating the core particles wherein a linker compound is introduced on the surface of the cores and the micelles include a nonionic surfactant consisting of a hydrophilic moiety containing at least two chains and a hydrophobic moiety containing at least one hydrocarbon chain.

In one embodiment of the present invention, the linker may be a click reaction-inducing compound.

In a further embodiment of the present invention, the linker may be an amphiphilic compound containing a hydrophobic moiety at one end thereof and a hydrophilic moiety bound with a click reactive material at the other end thereof.

The present invention also provides a method for producing nanoparticles for multiplex assays, including: providing fine iron oxide nanoparticle cores; modifying the core particles with a click reaction-inducing compound; providing a solution of micelles including a nonionic surfactant consisting of a hydrophilic moiety containing at least two chains and a hydrophobic moiety containing at least one hydrocarbon chain; dispersing the modified core particles in the micelle solution to render the modified core particles hydrophilic via ligand encapsulation; and binding a functionalizing material containing a click reactive moiety to the surface of the core particles to modify the surface of the core particles.

In one embodiment of the present invention, the nonionic surfactant may be used in 15- to 25-fold molar excess relative to the hydrophobic material on the surface of the cores for hydrophilization.

In a further embodiment of the present invention, the functionalizing material may be selected from the group consisting of disease targeting agents, chelating agents, fluorescent materials, and mixtures thereof.

In another embodiment of the present invention, the method may further include density gradient centrifugation.

According to a further aspect of the present invention, there is provided a method for producing nanoparticles for multiplex assays, including: providing fine iron oxide nanoparticle cores; providing a solution of micelles in which a nonionic surfactant consisting of a hydrophilic moiety containing at least two chains and a hydrophobic moiety containing at least one hydrocarbon chain is mixed with an amphiphilic material containing a functionalizing group; and dispersing the core particles in the micelle solution such that hydrolyzation of the core particles via ligand encapsulation and surface modification of the core particles with the functionalizing material are performed simultaneously.

In one embodiment of the present invention, the method may further include density gradient centrifugation.

The MRI T1 contrast agent based on ultrafine iron oxide nanoparticles according to the present invention can replace existing gadolinium MRI T1 contrast agents. In addition, the hydrophilization ensures the uniformity of the nanoparticles, enabling rapid distribution of the MRI T1 contrast agent in the blood and rapid excretion of the MRI T1 contrast agent from the body.

Furthermore, according to the platform for multiplex assays and the method for producing nanoparticles for multiplex assays, a functionalizing material containing a click reactive moiety is conjugated to the surface of an iron oxide nanoparticle platform to modify the surface of the nanoparticle platform and the surface-modified nanoparticle platform is hydrophilized by encapsulation with micelles.

### Brief Description of the Drawings

Fig. 1 explains the principle how an iron oxide nanoparticle of the present invention is hydrophilized.

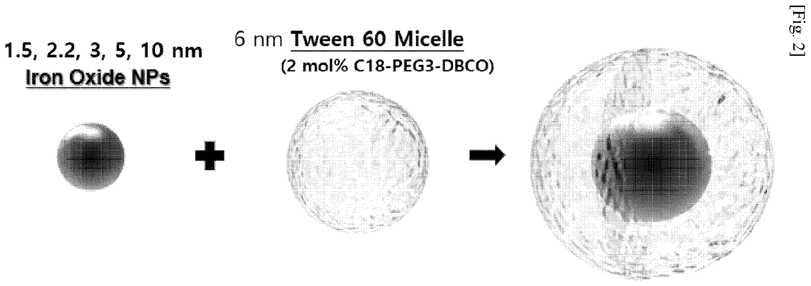

Fig. 2 schematically shows the reaction of hydrophilized iron oxide nanoparticles according to the present invention.

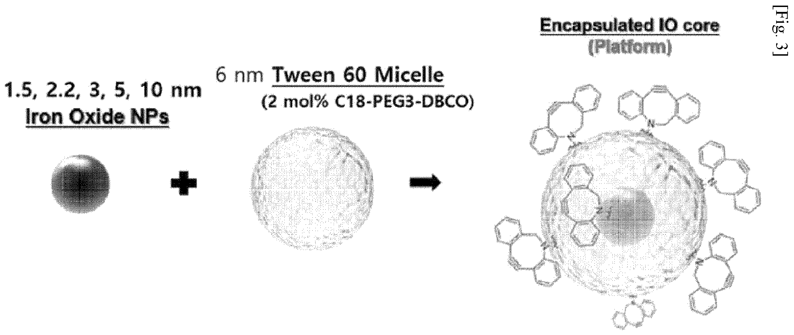

Fig. 3 schematically shows the application of a click reaction-inducing compound and the reaction of hydrophilized iron oxide nanoparticles according to the present invention.
Fig. 4 shows the storage stabilities of hydrophilized iron oxide nanoparticles, which were evaluated in Experimental Example 1.
Fig. 5 shows the storage stabilities of hydrophilized iron oxide nanoparticles in serum, which were evaluated in Experimental Example 1.

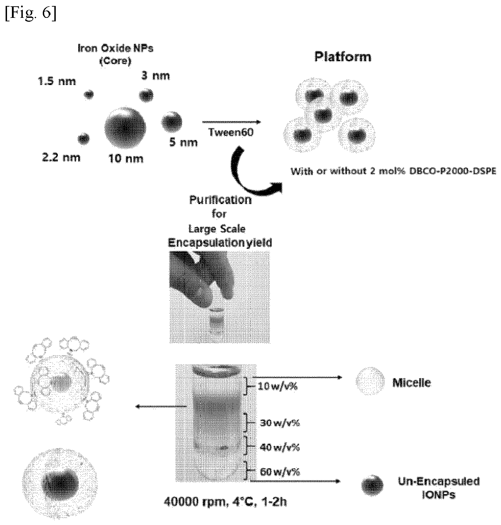

Fig. 6 shows a solution of hydrophilized iron oxide nanoparticles and remaining micelles, which were separated due to density differences to form layers.
Fig. 7 is a table showing the yields of hydrophilized iron oxide nanoparticles according to the present invention when prepared on a large scale.
Fig. 8 shows (A) the sizes of hydrophilized iron oxide nanoparticles according to the present invention when prepared on a large scale and (B) photographs of a solution containing the hydrophilized nanoparticles (B).
Fig. 9 shows images confirming the *in vivo* distributions of hydrophilized iron oxide nanoparticles according to the present invention.
Fig. 10 shows the results of radiolabeling, storage stability, and radiolabeling stability of hydrophilized iron oxide nanoparticles according to the present invention confirming the *in vivo* distributions of the hydrophilized nanoparticles.
Fig. 11 shows quantification data of PET and MRI of the great vessels using hydrophilized iron oxide nanoparticles of the present invention.
Fig. 12 is a photograph showing solid-phase hydrophilized iron oxide nanoparticles after removal of all liquid components by evaporation in Experimental Example 5.
Fig. 13 shows the results of DLS on hydrophilized iron oxide nanoparticles of the present invention that were redispersed a predetermined time after preparation in order to evaluate the applicability of the hydrophilized iron oxide nanoparticles to a kit.
Fig. 14 shows the results of an animal experiment using hydrophilized iron oxide nanoparticles of the present invention in order to determine the contrast effects of the hydrophilized nanoparticles in the liver, inferior vena cava (IVC), left ventricle (LV), aorta, kidney, and renal medulla of a rabbit after injection of the hydrophilized nanoparticles.
Fig. 15 shows the results of an animal experiment using hydrophilized iron oxide nanoparticles of the present invention in order to compare the contrast intensities of the hydrophilized nanoparticles in the liver, inferior vena cava (IVC), left ventricle (LV), aorta, kidney, and renal medulla of a rabbit after injection of the hydrophilized nanoparticles with those of a gadolinium-based contrast agent.

### Mode for Carrying out the Invention

Gadolinium and manganese are elements that do not naturally occur in the human body. Gadolinium and manganese remain in the body and cause side effects such as permanent deposition and scleroderma after use as materials for contrast agents. Iron is the central atom of hemoglobin, an important molecule that binds to oxygen in human red blood cells, and is one of the major elements constituting the human body. Iron deficiency anemia is caused by the lack of iron. Thus, iron is very unlikely to cause side effects in humans even when used as a material for a contrast agent. Particularly, iron oxide nanoparticles have been used clinically for diagnostic T2 contrast agents to diagnose liver cancer and are highly biocompatible materials for therapeutic agents for anemia as well as MRI contrast agents.

Development of ultrafine iron oxide nanoparticles for MRI T1 contrast agents requires a technique for synthesizing cores having a uniform size and a technique for hydrophilizing the core nanoparticles while maintaining their uniform size. The present invention provides an MRI T1 contrast agent based on ultrafine iron oxide nanoparticles that can replace existing gadolinium MRI T1 contrast agents. In addition, the present invention uses a hydrophilization technique for ensuring the uniformity of nanoparticles to enable rapid distribution of the MRI T1 contrast agent in the blood and angiography for at least 1 hour. The present invention will now be described in detail.

The term "ultrafine iron oxide nanoparticles" as used herein has the same meaning as the term "fine iron oxide nanoparticles". The term "hydrophilized iron oxide nanoparticles" as used herein has the same meaning as the term "hydrophilic iron oxide nanoparticles".

Specifically, the present invention provides a T1 contrast agent for magnetic resonance imaging including fine iron oxide nanoparticle cores and micelles encapsulating the core particles wherein the micelles include a nonionic surfactant consisting of a hydrophilic moiety containing at least two chains and a hydrophobic moiety containing at least one hydrocarbon chain.

The cores preferably have a diameter of 6 nm or less and a PDI of 0.2 or less. When the cores are controlled to a size of 6 nm or less, preferably 4 nm to 1 nm, more preferably 3 nm to 1.5 nm, the magnetic interaction between the iron ions distributed on the surface of the nanoparticles is diminished, with the result that the magnetic properties of the nanoparticles are changed to paramagnetism similar to that of gadolinium. When the magnetic properties of the nanoparticles are changed from superparamagnetism to paramagnetism, the interference of the T1 contrast effect with the T2 contrast effect disappears, enabling efficient use of the iron nanoparticles for the T1 contrast agent. The iron oxide nanoparticles have a diameter of 6 nm or less, preferably 4 nm to 1 nm, more preferably 3 nm to 1.5 nm, and a PDI of 0.2 or less, preferably 0.01 to 0.2, more preferably 0.1 to 0.2. Within these ranges, a better T1-weighted contrast effect can be exhibited and the size of the nanoparticles can be maintained constant during storage.

The cores can be prepared by reacting an iron complex including iron as a central iron atom and C₄-C₂₅ organic acid groups (carboxylates) as ligands bound to the central atom, a C₄-C₂₅ fatty acid, and a C₄-C₂₅ aliphatic alcohol or amine. Specifically, the core nanoparticles can be prepared by the following procedure. First, the iron complex, the fatty acid, and the aliphatic alcohol (or aliphatic amine) as raw materials are mixed. The mixture is allowed to react. The reaction temperature is raised from room temperature to 150 to 350 °C at a rate of 5 °C/min or more and is then maintained at 150 to 350 °C for 5 to 60 minutes. The size of the cores can be controlled by varying the molar ratio of the C₄-C₂₅ fatty acid and the C₄-C₂₅ aliphatic alcohol or amine as raw materials. Since the fine iron oxide nanoparticle cores have a diameter of 6 nm or less, a uniform size, a PDI of 0.2 or less, and a uniform distribution, a constant contrast effect can be attained.

Preferably, the iron complex includes an iron atom and C₄-C₂₅ organic acid groups as ligands bound to the central iron atom. Examples of the ligands include stearic acid, oleic acid, linoleic acid, palmitic acid, palmitoleic acid, myristic acid, lauric acid, arachidonic acid, and behenic acid. Preferably the iron complex may be iron oleate. The C₄-C₂₅ fatty acid may be, for example, stearic acid, oleic acid, linoleic acid, palmitic acid, palmitoleic acid, myristic acid, lauric acid, arachidonic acid, ricinoleic acid or behenic acid. The C₄-C₂₅ aliphatic alcohol may be, for example, stearyl alcohol (octadecanol), oleyl alcohol, linoleyl alcohol, hexadecanol, palmitoleyl alcohol, tetradecanol, dodecanol, arachidonyl alcohol, eicosanol, docosanol or hexadecanediol. The C₄-C₂₅ aliphatic amine may be, for example, stearylamine (octadecylamine), oleylamine, hexadecylamine, palmitoleylamine, tetradecylamine, dodecylamine or arachidonylamine. Most preferably, the fatty acid is oleic acid, the aliphatic alcohol is oleyl alcohol, and the aliphatic amine is oleylamine.

The use of the iron oxide nanoparticles for the T1 contrast agent requires hydrophilization of the fine iron oxide nanoparticles for dispersion in a solution because the surface of the iron oxide nanoparticle cores is hydrophobic, as well as control over the size of the fine iron oxide nanoparticles. The hydrophilization may be accomplished by a suitable process, for example, substitution or coating. The substitution process refers to a process for exchanging the hydrophobic moiety on the surface of the nanoparticles with a hydrophilic material and the coating process refers to a process for covering the surface of the nanoparticles with a hydrophilic material (e.g., a polymer or silica). According to a commonly used substitution process, ligands are substituted with a material such as polyethylene glycol-phosphate (PO-PEG) in an organic solvent, followed by redispersion in water as a new dispersion solvent. PO-PEG is used in a large amount for hydrophilization. Purification of iron oxide nanoparticles is essential as an additional process for functionalization but it causes problems in terms of yield and process stability. Another problem is that aggregation may occur in the multi-step reaction depending on the polarity of terminal functional groups. Further, many batch-to-batch variations are inevitable until the final material is obtained. It is also known that the hydrated radius becomes large compared to the core size during hydrophilization. In conclusion, the conventional substitution process suffers from difficulty in hydrophilizing nanoparticles while maintaining the ultrafine size of the nanoparticles.

These problems are solved by encapsulation of core particles with micelles to hydrophilize the surface of the core particles. The micelles include a nonionic surfactant consisting of a hydrophilic moiety containing at least two chains and a hydrophobic moiety containing at least one hydrocarbon chain. According to the present invention, the encapsulation of the small and uniform core particles with the micelles enables stable hydrophilization of the nanoparticles without aggregation, prevents a significant increase in hydrated radius even after hydrophilization, and allows the core particles to have a PDI of 0.2 or less. Thus, the nanoparticles are suitable for use in the T1 contrast agent and can be prepared on a large scale.

The nonionic surfactant may have an **A-O-C(O)-B** structure (wherein **A** is a hydrophilic moiety and **B** is a hydrophobic moiety). The hydrophilic moiety may include two or more chains, each of which has one or more terminal -OH groups and includes - O-(CH₂)ₙ- or a C₃-C₅ alicyclic hydrocarbon group. The two or more of these may be optionally bonded to each other to form a chain structure. The chain structure may be branched such that the number of terminal groups is 2 or more, preferably 2 to 10, more preferably 2 to 8. The terminal groups may be -OH groups. When the number of the terminal groups is in the range defined above, improved hydrophilicity can be achieved.

Each of the alicyclic hydrocarbon groups is preferably interrupted by an oxygen, sulfur or nitrogen atom capable of hydrogen bonding with a solvent. As another example, one or more double bonds may be present in each alicyclic hydrocarbon ring.

In each of the -O-(CH₂)ₙ blocks, n is an integer ranging from 1 to 10, preferably from 2 to 8, more preferably from 2 to 5. Within this range, improved hydrophilicity can be achieved. Each of the -O-(CH₂)ₙ blocks may be, for example, polyethylene glycol, polypropylene glycol or polybutylene glycol.

The -O-(CH₂)ₙ blocks may account for at least 80 wt% or at least 90 wt% of the moiety **A.** The moiety **A** may include a C₁₀-C₆₀ hydrocarbon group, preferably a C₂₀-C₅₀ hydrocarbon group, in its chain structure. When the number of carbon atoms in the hydrocarbon group is within the range defined above, good dispersion stability of the nanoparticles in an aqueous solution can be ensured due to the steric hindrance effect and high hydrophilicity.

The hydrophobic moiety **B** may be at least one hydrocarbon chain, preferably a C₆-C₃₀ hydrocarbon chain, preferably a C₁₀-C₂₀ hydrocarbon chain. The hydrocarbon chain may include an aliphatic hydrocarbon group having 2 or more carbon atoms (for example, alkyl, alkenyl or alkynyl group), an aromatic hydrocarbon group having 6 or more carbon atoms (for example, phenyl, naphthyl or aralkyl group) or an alicyclic hydrocarbon group having 5 or more carbon atoms (for example, cyclohexyl or norbornenyl group) in the middle or at one end. Alternatively, the hydrocarbon chain may include a branched hydrocarbon chain. The hydrocarbon chain may optionally include one or more double bonds in the middle. Specific examples of such hydrocarbon chains may include monolaurate, monopalmitate, monostearate, and monooleate.

The nonionic surfactant is preferably a polysorbate surfactant. Specifically, the polysorbate surfactant may be Polysorbate 20 (polyoxyethylene (20) sorbitan monolaurate, Tween 20), Polysorbate 40 (polyoxyethylene (20) sorbitan monopalmitate, Tween 40), Polysorbate 60 (polyoxyethylene (20) sorbitan monostearate, Tween 60) or Polysorbate 80 (polyoxyethylene (20) sorbitan monooleate, Tween 80).

Polysorbate 60 (polyoxyethylene (20) sorbitan monostearate, Tween 60) as the nonionic surfactant has a structure represented by Formula 1: 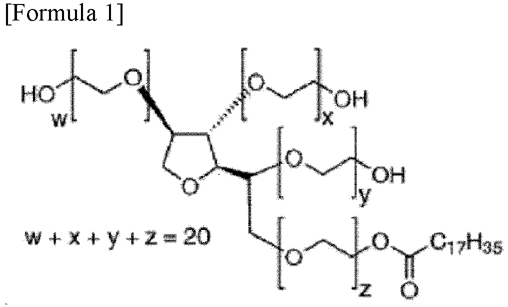 wherein w, x, y, and z are integers.

The principle of dispersion stabilization can be considered to be due to the hydrophobic-hydrophobic interaction between the hydrophobic chain of the fatty acid on the surface of the nanoparticles and the hydrophobic groups of the nonionic surfactant, the hydrogen bonding between the hydrophilic groups of the nonionic surfactant and surrounding water molecules, and the steric hindrance effect.

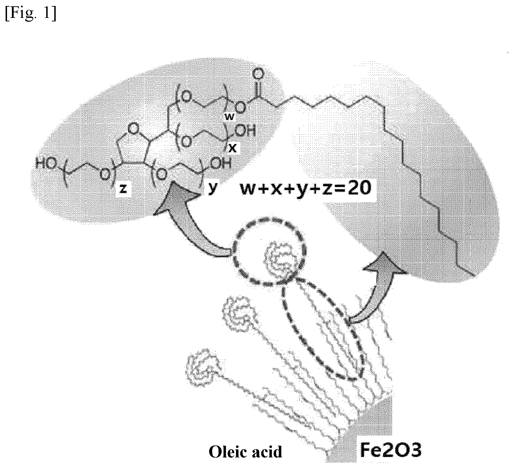

Fig. 1 is a diagram showing the principle of dispersion stabilization of the iron oxide nanoparticles with a polysorbate surfactant as the nonionic surfactant. Referring to Fig. 1, the hydrophobic group of the polysorbate nonionic surfactant interacts and forms a bond with the hydrophobic group of the fatty acid present on the surface of the iron oxide nanoparticles. The hydrophilic group of the nonionic surfactant interacting with surrounding water molecules basically has polyalkylene glycol blocks, preferably 2 or more polyalkylene glycol blocks, more preferably 3 or more polyalkylene glycol blocks, each of which has a terminal -OH group. The polyalkylene glycol blocks form a bulky terminal hydrophilic group. For example, Tween 60 of Formula 1 has a structure in which a branched hydrophilic chain having three terminal -OH groups and a C₁₇ hydrophobic chain are bonded to an ester group. As a result, the micelle structures can be maintained stable without aggregation even after encapsulation of the iron oxide nanoparticles due to the steric hindrance effect and high hydrophilicity.

The cores can be encapsulated with the micelles by adding the core particles to a micelle solution containing the nonionic surfactant and uniformly dispersing the core particles by sonication. The nonionic surfactant is preferably used in 15- to 25-fold molar excess relative to the hydrophobic material on the surface of the cores. If the amount of the nonionic surfactant is less than the lower limit (*i.e.* the amount of the hydrophilizing material is small), hydrophilization proceeds in a state in which the nanoparticles are not sufficiently dispersed and aggregate, resulting in low yield. Meanwhile, if the amount of the nonionic surfactant exceeds the upper limit, the nanoparticles may increase in diameter or may aggregate during hydrophilization, which affects subsequent separation of the nanoparticles. Therefore, the amount of the nonionic surfactant added during hydrophilization of the cores is an important factor and needs to be appropriately adjusted to the range defined above depending on the size of the nanoparticles. For example, since the surface area of the nanoparticles increases with decreasing size of the nanoparticles, the number of the hydrophobic groups of the fatty acid on the surface of the cores increases. It is thus necessary to add a larger amount of the hydrophilizing material reacting with the fatty acid.

The hydrophilized nanoparticles have an overall diameter of 10 nm or less, preferably 8 nm to 1 nm, more preferably 8 nm to 1.5 nm, and a PDI of 0.2 or less, preferably 0.01 to 0.2, more preferably 0.1 to 0.2. If the diameter and PDI exceed the respective upper limits, a significant T1-weighted contrast effect is not expected and a uniform distribution in the human body is difficult to obtain. The contrast agent of the present invention exhibits a contrast effect similar to that of a gadolinium-based contrast agent, is rapidly distributed in the blood, can be used to image blood vessels for at least 1 hour, and can be excreted through the kidneys and liver after contrast.

The present invention also provides a platform for multiplex assays with a magnetic resonance imaging T1 contrast effect, including fine iron oxide nanoparticle cores and micelles encapsulating the core particles wherein a linker compound is introduced on the surface of the cores and the micelles include a nonionic surfactant consisting of a hydrophilic moiety containing at least two chains and a hydrophobic moiety containing at least one hydrocarbon chain.

Surface modification of the cores with a click compound provides a platform for multiplex assays capable of multi-labeling. The linker is preferably a click reaction-inducing compound, more preferably an amphiphilic compound containing a hydrophobic moiety at one end thereof and a hydrophilic moiety bound with a click reactive material at the other end thereof. The linker may be, for example, a compound into which a single-chain (C₁₂-C₁₈) functional group, such as an aza-dibenzocyclooctyne compound (for example, ADIBO or DBCO). Examples of such functional groups include ADIBO-PEG4-C18, ADIBO-PEG2000-DSPE, DBCO-PEG4-C18, DBCO-PEG2000-DSPE, DBCO-SA, N3-PEG4-C18, and N3-PEG2000-DSPE. Particularly, the micelles using DBCO-PEG2000-DSPE reduce the uptake of the nanoparticles by the liver and enable circulation of the nanoparticles in the blood for up to 2 hours.

The linker compound may be introduced on the core surface by mixing 1 to 15 mol%, preferably 1 to 10 mol%, more preferably 1 to 8 mol% of the nanoparticles dispersed in an organic solvent with a click compound having a functional group. The click chemistry reaction is carried out under simple conditions, does not require high temperature, catalyst, acid-base conditions, etc., and leads to very high yield and conversion rate.

The present invention also provides a method for producing nanoparticles for multiplex assays, including: providing fine iron oxide nanoparticle cores; modifying the core particles with a click reaction-inducing compound; providing a solution of micelles including a nonionic surfactant consisting of a hydrophilic moiety containing at least two chains and a hydrophobic moiety containing at least one hydrocarbon chain; dispersing the modified core particles in the micelle solution to render the modified core particles hydrophilic via ligand encapsulation; and binding a functionalizing material containing a click reactive moiety to the surface of the core particles to modify the surface of the core particles.

According to the method of the present invention, the surface of fine iron oxide nanoparticle cores is modified with a click reaction-inducing compound, hydrophilized, and bound with a functionalizing material containing a click reactive moiety to produce nanoparticles for multiplex assays in which various functional groups are introduced. The hydrophilization is preferably performed using the nonionic surfactant in 15- to 25-fold molar excess relative to the hydrophobic material on the surface of the cores. The functionalizing material may be selected from the group consisting of disease targeting agents, chelating agents, fluorescent materials, and mixtures thereof.

It is desirable that the method further includes density gradient centrifugation. The density gradient centrifugation is a process in which a sample is placed in a density gradient solution and centrifuged to obtain desired particles. The density gradient centrifugation enables efficient separation of particles with different sizes, unlike column fractionation with low yield and fractional centrifugation causing co-precipitation of particles with different sizes. The density gradient centrifugation is a process for separating particles in high purity on the basis of density. The density gradient centrifugation uses a density gradient solution to separate hydrophilized nanoparticles and non-hydrophilized nanoparticles, selectively separate nanoparticles having a desired size and PDI, and purify a large amount of hydrophilized nanoparticles.

In the method of the present invention, the steps may be carried out simultaneously rather than sequentially.

The present invention also provides a method for producing nanoparticles for multiplex assays, including: providing fine iron oxide nanoparticle cores; providing a solution of micelles in which a nonionic surfactant consisting of a hydrophilic moiety containing at least two chains and a hydrophobic moiety containing at least one hydrocarbon chain is mixed with an amphiphilic material containing a functionalizing group; and dispersing the core particles in the micelle solution such that hydrolyzation of the core particles via ligand encapsulation and surface modification of the core particles with the functionalizing material are performed simultaneously. According to the method of the present invention, the surface of the core nanoparticles can be modified with a hydrophilizing material and a functionalizing material by a one-step process in which a click reaction-inducing compound and the functionalizing material are added to and mixed with the micelle solution during hydrophilization of the cores, without the need to separately perform the steps. As such, the method of the present invention is carried out in a simple way, enabling the synthesis of nanoparticles for multiplex assays on a large scale.

According to the method of the present invention, the fine iron oxide nanoparticle cores encapsulated with the micelles are fine and uniform in size and have a uniform PDI of 0.2 or less. The cores are made hydrophilic by encapsulation with the micelles including nonionic surfactant. The encapsulation of the cores using the nonionic surfactant makes the size of the nanoparticles uniform without a significant increase in hydrated diameter even after hydrophilization and allows the cores to have a uniform PDI of 0.2 or less. In addition, the nanoparticles hardly aggregate both *in vitro* and *in vivo,* indicating their good storage stability. The T1 contrast agent for magnetic resonance imaging according to the present invention is a novel one based on fine iron oxide nanoparticles that can replace conventional gadolinium-based T1 contrast agents. The T1 contrast agent based on fine iron oxide nanoparticles according to the present invention is harmless to humans, is rapidly distributed in the blood, and has a uniform size, ensuring its uniform contrast effect. In addition, the T1 contrast agent of the present invention enables image observation for at least 1 hour to up to 2 hours. The hydrophilization of the uniformly sized (100 nm or less) nanoparticles reduces the uptake of the nanoparticles into the reticuloendothelial system of the liver, with the result that the T1 contrast agent stays in the bloodstream for a prolonged time, does not accumulate in the human body, and is excreted through the kidneys and liver. Therefore, the T1 contrast agent of the present invention avoids the problems encountered in conventional gadolinium-based contrast agents.

As described above, the hydrophilization is effective in reducing the uptake of the nanoparticles into the reticuloendothelial system of the liver. The fact that the size of nanoparticles determines organs where uptake of the nanoparticles occurs and the distribution of the nanoparticles in the human body is well known in the art. Nanoparticles having a size of 50 nm or more are rapidly accumulated *in vivo* by the Kupffer cells in the liver. Even small core nanoparticles tend to aggregate unless they are sufficiently dispersed. This aggregation increases the uptake of the core nanoparticles into the reticuloendothelial system.

The present invention also provides a platform for multiplex assays and a method for producing nanoparticles for multiplex assays in which cores are surface modified with a hydrophilizing material and a functionalizing material, achieving T1 contrast effects, and various functional groups are introduced. According to the present invention, the surface of nanoparticle cores can be modified with a hydrophilizing material and a functionalizing material by a one-step process in which a click reaction-inducing compound and the functionalizing material are added to and mixed with a micelle solution during hydrophilization of the cores. As such, the method of the present invention is carried out in a simple way. In addition, nanoparticles can be separated and purified in a simple manner and can be prepared in high yield. Therefore, the method of the present invention is suitable for large-scale production of nanoparticles for multiplex assays.

The following examples may be changed into several other forms and are not intended to limit the scope of the present invention. These examples are provided to more fully explain the present invention to those skilled in the art.

### Example 1-1: Preparation of 3 nm ultrafine iron oxide nanoparticles

1.8 g (2 mmol) of iron oleate, 0.57 g (2 mmol) of oleic acid, and 1.61 g (6 mmol) of oleyl alcohol were mixed with 10 g of diphenyl ether. The mixture was placed in a round-bottom flask. The flask was evacuated to a vacuum at 80 °C for ∼1 h to remove air. Thereafter, an inert environment was created under a flow of argon. The reaction was carried out while raising the temperature to 250 °C at a rate of 10 °C/min. As the reaction proceeded, the reaction mixture turned black in color. The reaction at 250 °C for 30 min afforded 3 nm nanoparticles, which was rapidly cooled and washed with excess acetone. The resulting precipitate was dispersed in chloroform or hexane as an organic solvent.

### Example 1-2: Preparation of 5 nm ultrafine iron oxide nanoparticles

1.8 g (2 mmol) of iron oleate and 0.28 g (1 mmol) of oleic acid were mixed with 10 g of octadecene. The mixture was placed in a round-bottom flask. The flask was evacuated to a vacuum at 80 °C for ∼1 h to remove air. Thereafter, an inert environment was created under a flow of argon. The reaction was carried out while raising the temperature to 317 °C at a rate of 10 °C/min. As the reaction proceeded, the reaction mixture turned black in color. The reaction at 317 °C for 30 min afforded ∼5 nm nanoparticles, which was rapidly cooled and washed with excess acetone. The resulting precipitate was dispersed in chloroform or hexane as an organic solvent.

### Example 1-3: Preparation of 10 nm ultrafine iron oxide nanoparticles

1.8 g (2 mmol) of iron oleate and 0.28 g (1 mmol) of oleic acid were mixed with 10 g of octadecene. The mixture was placed in a round-bottom flask. The flask was evacuated to a vacuum at 80 °C for ∼1 h to remove air. An inert environment was created under a flow of argon. The reaction was carried out while raising the temperature to 315 °C at a rate of 10 °C/min. As the reaction proceeded, the reaction mixture turned black in color. The reaction at 315 °C for 30 min afforded ∼10 nm nanoparticles, which was rapidly cooled and washed with excess acetone. The resulting precipitate was dispersed in chloroform or hexane as an organic solvent.

### Example 2: Hydrophilization of the ultrafine iron oxide nanoparticles

The ultrafine iron oxide nanoparticles (IONPs) prepared in Examples 1-1 to 1-3 were hydrophilized through the following steps.
(1) Solid IONPs: The solid IONPs synthesized in each of Examples 1-1 to 1-3 were dried, weighed, and dispersed in chloroform to a concentration of 20 mg/mL.
(2) The 1.5, 2.2, 3, 5, and 10 nm ultrafine iron oxide nanoparticles (IONPs) were hydrophilized with Tween 60 and oleic acid, which were used in the ratio shown in Table 1.

**[Table 1]**

| Ratio | Value |
|---|---|
| T60/OA | 19.5625 (∼20-fold) |

In Table 1, "OA" is the number of moles of oleic acid on the surface of the nanoparticles and "T60" is the number of moles of Tween 60. The molar ratio "T60/OA" was ∼20 and the same applied to all nanoparticles. The calculated ratio shows that a smaller size of the nanoparticles leads to a larger amount of OA relative to the dry weight of the IONPs, indicating that a larger amount of Tween 60 should be used.

The amount of OA introduced on the surface of one of the iron nanoparticle cores is calculated by a factor *f*, which can be determined from the experimental values for the nanoparticles synthesized in each of Examples 1-1 to 1-3. The/values are shown in Table 2.

**[Table 2]**

| | | | | | |
|---|---|---|---|---|---|
| *f* | 1.5 nm | 2.2 nm | 3 nm | 5 nm | 10 nm |
| M_{core}/Mₜₒₜₐₗ | 0.398936 | 0.493274 | 0.570342 | 0.688705 | 0.815661 |

In Table 2, "M_{core}" represents the mass of the iron component relative to the total dry mass of all iron nanoparticles and "Mₜₒₜₐₗ" represents the total dry mass. That is, the mass of pure iron can be calculated by multiplying the total mass by the factor *f*. The mass of the surface-attached ligands can be calculated by subtracting the mass of pure iron from the total mass to determine the number of moles of OA.
(3) A micelle solution was prepared using the amount of Tween60 determined in (2). Tween 60 was dissolved in distilled water to prepare 500-1000 ml of a 5-10% (v/v) aqueous solution of Tween 60 and sonicated at 60 °C for 10 min until the solution was transparent. Alternatively, the micelle solution may be prepared with stirring in a thermostat at 60°C.
(4) The nanoparticles were hydrophilized with the 5-10% (v/v) micelle solution and were uniformly dispersed using a sonicator (Ultra-sonicator). When 100 mg (5 mL) of the 3 nm nanoparticles were dispersed in chloroform to a concentration of 20 mg/mL, added to 40 mL of the 10% (v/v) micelle solution, and sonicated at 60 °C for 10 min, the opaque brown (latte color) suspension became clear and turned transparent brown (americano color). At that time, the solution where the reaction was taking place was placed on a stirrer at 60 °C, followed by stirring for additional 10 min to completely remove the possibly remaining organic solvent.

### Example 3: Surface modification of the hydrophilized ultrafine iron oxide nanoparticles with click compound

In this example, the hydrophilized ultrafine iron oxide nanoparticles were surface modified with a click compound through the following steps.
(1) Solid IONPs: The solid IONPs synthesized in each of Examples 1-1 to 1-3 were dried, weighed, and dispersed in chloroform to a concentration of 20 mg/mL.
(2) The 1.5, 2.2, 3, 5, and 10 nm IONPs were hydrophilized with Tween 60 and oleic acid, which were used in the ratio shown in Table 3, and surface modified with functional groups of a click compound (DBCO) whose ratio to Tween 60 is shown in Table 3.

**[Table 3]**

| Ratio | Value |
|---|---|
| T60/OA | 19.5625 (∼20-fold) |
| DBCO/T60 | 0.20754717 |

In Table 3, "OA" is the number of moles of oleic acid on the surface of the nanoparticles and "T60" is the number of moles of Tween 60. The molar ratio "T60/OA" was ∼20 and the same applied to all nanoparticles. The calculated ratio shows that a smaller size of the nanoparticles leads to a larger amount of OA relative to the dry weight of the IONPs, indicating that a larger amount of Tween 60 should be used.

1-10 mol% DSPE-PEG2000-DBCO was used for functionalization and surface modification with a click compound and encapsulation with micelles. The number of moles of OA was calculated as described in Example 2.
(3) A micelle solution was prepared using the amounts of Tween60 and the click compound determined in (2). Tween 60 was dissolved in distilled water to prepare 500-1000 ml of a 5-10% (v/v) aqueous solution of Tween 60, 1-10 mol% DSPE-PEG2000-DBCO was added to the micelle solution, and sonication was performed at 60 °C for 10 min until the solution was transparent. Alternatively, the micelle solution may be prepared with stirring in a thermostat at 60°C.
(4) The nanoparticles were hydrophilized with the 5-10% (v/v) micelle solution and were uniformly dispersed using a sonicator (Ultra-sonicator). When 100 mg (5 mL) of the 3 nm nanoparticles were dispersed in chloroform to a concentration of 20 mg/mL, added to 40 mL of the 10% (v/v) micelle solution, 2 mol% DSPE-PEG2000-DBCO relative to Tween60 was added to the micelle solution, and sonicated at 60 °C for 10 min, the opaque brown (latte color) suspension became clear and turned transparent brown (americano color). At that time, the solution where the reaction was taking place was placed on a stirrer at 60 °C, followed by stirring for additional 10 min to completely remove the possibly remaining organic solvent.

### Experimental Example 1: Evaluation of size-dependent in vitro and in vivo stabilities of the hydrophilized iron oxide nanoparticles

In this experimental example, the size-dependent *in vitro* and *in vivo* stabilities of the hydrophilized iron oxide nanoparticles prepared in Example 2 were evaluated.

The hydrodynamic sizes of the hydrophilized nanoparticles were analyzed using a DLS instrument. The polydispersity index (PDI) was used as a measure of uniformity. Specifically, after storage in distilled water at room temperature for a specific period of time, the sizes of the 1.5 nm, 2.2 nm, 3 nm, 5 nm, and 10 nm hydrophilized iron oxide nanoparticles were again measured to determine whether their *in vitro* stabilities were maintained. In addition, the same amounts of the hydrophilized nanoparticles were dispersed in human serum and kept refrigerated. A determination was made as to whether the hydrophilized nanoparticles were precipitated during storage.

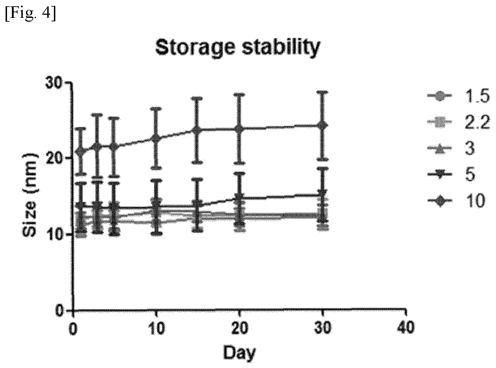

The stabilities during storage were tested for ∼1 month. The results are shown in Fig. 4. The small-sized nanoparticles were very stable but the 10 nm nanoparticles showed a tendency to gradually increase in size, as confirmed in Fig. 4. There results were believed to be because the larger size led to better precipitation.

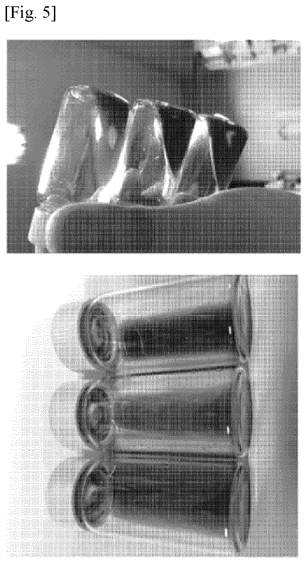

As confirmed in Fig. 5, even after the 2.2, 3, and 5 nm nanoparticles were dispersed in human serum and stored for ∼20 days, no precipitation or aggregation was observed with naked eyes.

The above results revealed that the hydrophilized iron oxide nanoparticles prepared by the inventive method were highly stable *in vitro* and *in vivo.*

### Experimental Example 2: Separation and purification of the hydrophilized iron oxide nanoparticles

In this experimental example, hydrophilized nanoparticles and micelles were separated and purified from the clear solution after hydrophilization in Example 2. The separation was performed by density gradient centrifugation.

Specifically, the clear solution was centrifuged at 15000 rpm and 4 °C for 1 h. As a result, nanoparticles that did not participate in hydrophilization and non-hydrophilized nanoparticles were settled down and formed a sticky sludge at the bottom. The above centrifugation conditions were applied to the ≥ 5 nm hydrophilized iron oxide nanoparticles. Alternatively, the < 5 nm hydrophilized iron oxide nanoparticles were centrifuged at 40000 rpm and 4 °C for 1-2 h.

Purification was performed using iodixanol (Opti-Prep) for centrifugation. When 10%, 30%, 40%, and 60% (w/v) iodixanol solutions (Opti-solution) were used, band-like layers were formed in the solutions due to the density differences (Fig. 5). When the actual PDI value was higher than expected after separation, a portion of the solution was dialyzed to completely remove iodixanol (Opti-Prep) before use. Micelles that did not participate in hydrophilization were not removed by dialysis.

### Experimental Example 3: Large-scale preparation and purification of the hydrophilized iron oxide nanoparticles

In this experimental example, large-scale hydrophilization was performed for medium and large animal experiments or to evaluate the clinical and industrial applications of the hydrophilized iron oxide nanoparticles.

The size of the core nanoparticles before encapsulation was fixed to 3 nm, followed by repeated hydrophilization under the same conditions to confirm the hydrophilization yield and to evaluate the size after hydrophilization. The same method as previously used was carried out in quintuplicate while strictly keeping the T60/OA ratio shown in Tables 1 and 3.

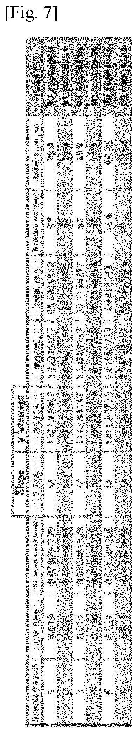

The results are shown in Fig. 7. As can be seen in Fig. 7, high yields of > 90%, on average, were obtained, indicating that the present invention provides a very effective hydrophilization protocol. Particularly, since the data of each round showed very high and similar yields even though hydrophilization was performed at different scales (using different amounts of the starting cores), which was judged to provide a great advantage for the preparation of hydrophilized iron oxide nanoparticles on an industrial scale in the future.

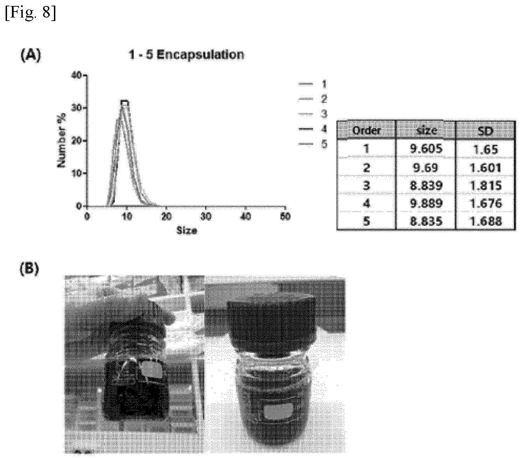

All hydrophilized nanoparticles were analyzed by DLS. As a result, not only the yields but also the sizes after hydrophilization were almost uniform ((A) of Fig. 8). The 3 nm hydrophilized nanoparticles prepared on a large scale are shown in (B) of Fig. 8.

### Experimental Example 4: Confirmation of size-dependent in vivo distributions of the hydrophilized iron oxide nanoparticles

In this experimental example, size-dependent *in vivo* distributions of the hydrophilized iron oxide nanoparticles were confirmed. To this end, the *in vivo* distributions of the hydrophilized iron oxide nanoparticles having different sizes were imaged and the reliability of the images was evaluated by nuclear medicine quantification.

The 2.2, 3, and 5 nm hydrophilized iron oxide nanoparticles prepared in Example 2 were stored for ∼2 months and experiments were performed in triplicate (n=3) or more. In each experiment, the hydrophilized iron oxide nanoparticles were labeled with Cu-64. Following the isotope protocol, the experiments were conducted at intervals of 2 weeks.

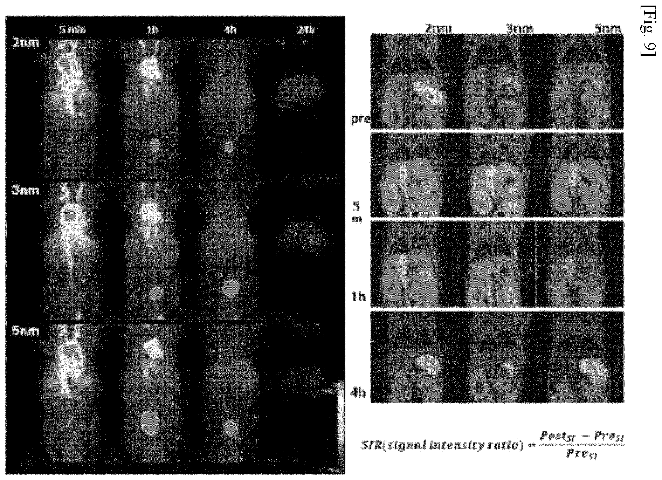

The experimental results are shown in Fig. 9. As confirmed in Fig. 9, the MRI images and the PET images were very well fitted, demonstrating that the hydrophilization material was circulated while remaining well attached to the isotopically labeled core surface.

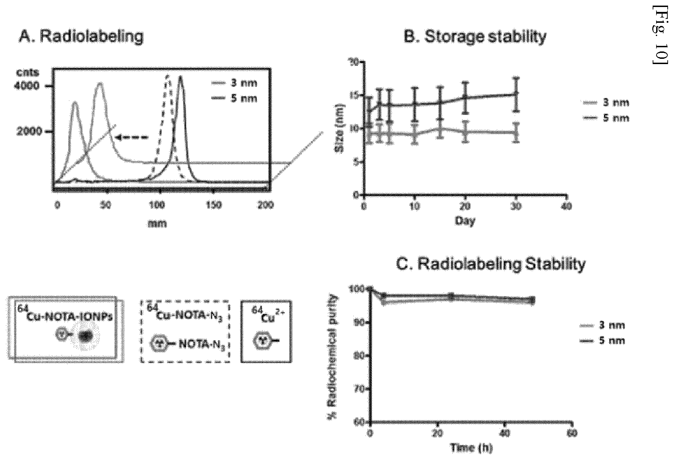

The isotopic labeling was performed by TLC. This labelling is also called radiolabeling. A schematic diagram and data of the isotopic labeling are shown in Fig. 10. Storage stability is a measure of whether the size of the hydrophilized nanoparticles is maintained (up to 4 weeks) even after isotopic labeling. At this time, radiolabeling stability was also confirmed.

In these experiments, similar PET/MRI data were confirmed even after storage for a total of 2 months. Although not shown, the hydrophilization material was stably bound to the core material without being separated for ∼1 year after preparation of the hydrophilized nanoparticles.

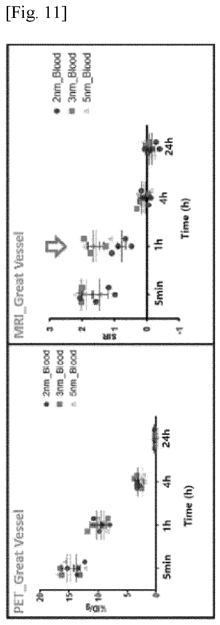

The quantification data shown in Fig. 11 reveal that the PET signals at each time point showed a similar trend to the MRI signals.

### Experimental Example 5: Evaluation of the applicability of the hydrophilized iron oxide nanoparticles to kits

In this experimental example, the applicability of the hydrophilized iron oxide nanoparticles to kits was evaluated. To this end, after the nanoparticles in an aqueous solution were dried under vacuum and redispersed, their size and PDI values were compared.

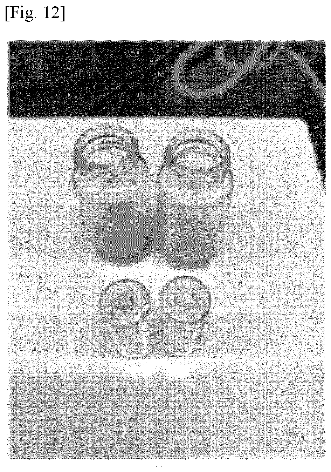

The 3 nm hydrophilized nanoparticles prepared in Example 2 were partially diluted and transferred to a small vial. The aqueous solution was evaporated to remove all liquid components. As can be seen from Fig. 12, clear circular bands were formed and a solid phase was formed at the bottom of the small vial. The solid phase was redispersed in distilled water (DW) and transferred to a larger vial. The size and PDI of the hydrophilized iron oxide nanoparticles were evaluated by DLS.

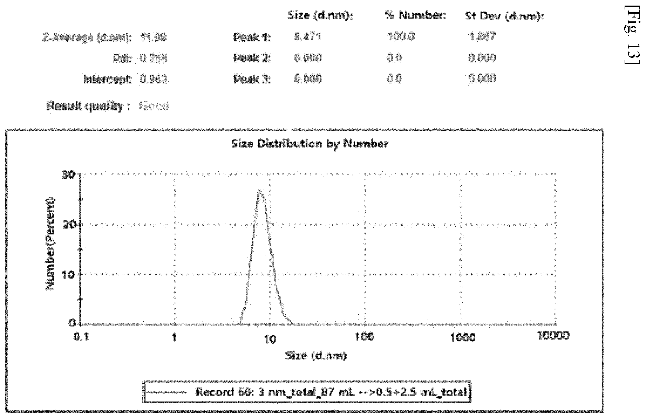

0.5 mL was taken from the 3 nm hydrophilized nanoparticles (a total of 87 mL), dried and redispersed in 2.5 mL of distilled water (DW). Fig. 13 shows the results of DLS on the hydrophilized nanoparticles. The size of the hydrophilized nanoparticles was not almost changed from that before redispersion. The hydrophilized nanoparticles were found to have a PDI of 0.2. The Z-average was also very close to the original average value, demonstrating that the same physical properties would be maintained even after redispersion.

### Experimental Example 6: Animal (rabbit) experiments on pharmacological action

In this experimental example, after administration of the 3 nm hydrophilized iron oxide nanoparticles and gadolinium to rabbits, contrast effects in the liver, inferior vena cava (IVC), left ventricle (LV), aorta, kidney, and renal medulla of the rabbits were evaluated.

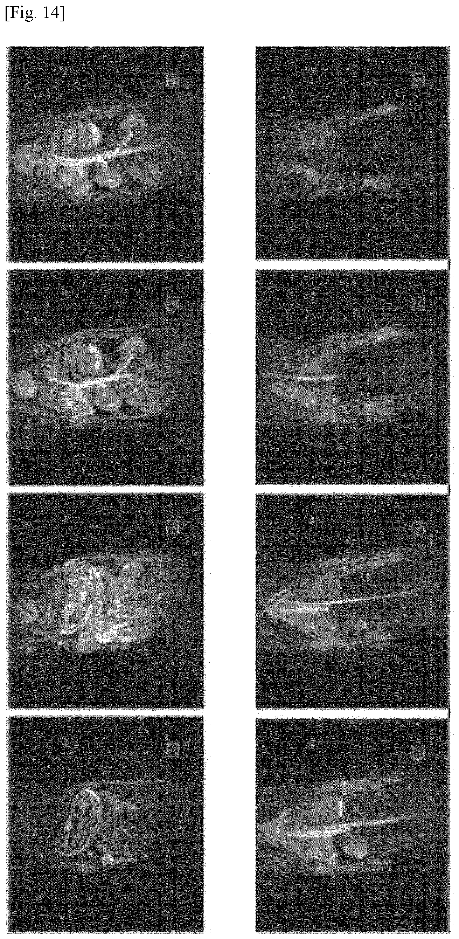

As shown in Fig. 14, contrast-enhanced MRI using the 3 nm hydrophilized iron oxide nanoparticles showed about half the contrast-enhancement effect of gadolinium. That is, the top MRI images, where circular heart parts and blood vessels appeared bright, revealed that the T1 effect of the hydrophilized nanoparticles did not disappear immediately after administration, unlike the existing contrast agent, and the hydrophilized nanoparticles were circulated well in the bloodstream.

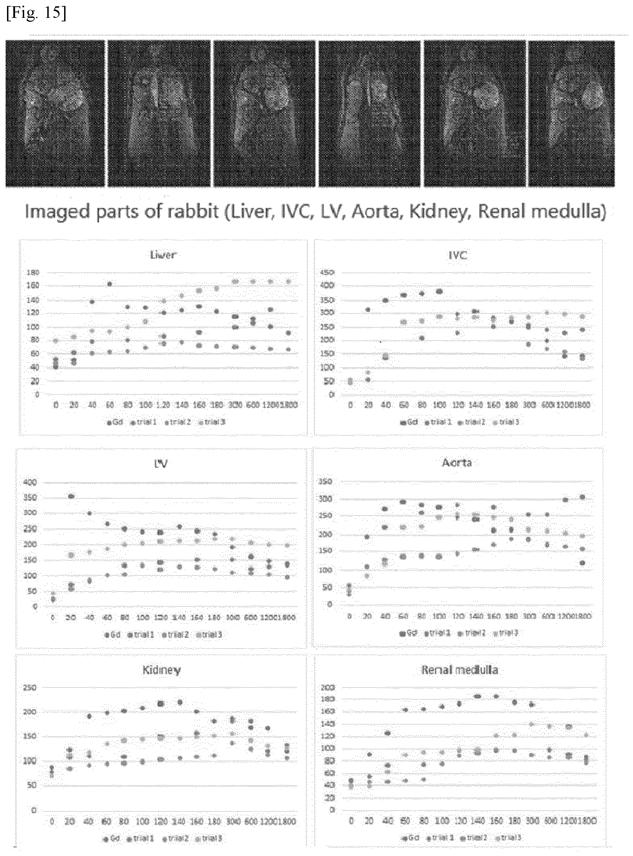

Trials 1, 2 and 3 in in Fig. 15 show the results obtained after administration of the 3 nm hydrophilized iron oxide nanoparticles three times. Here, the X-axis represents time (sec) and the Y-axis represents MRI signal. As can be seen from Fig. 15, the gadolinium contrast agent showed strong signals in the blood vessels at the initial stage of injection. Thereafter, the gadolinium contrast agent was distributed in the organs and excreted through the kidneys. In contrast, the hydrophilized iron oxide nanoparticles were distributed in the blood vessels and hardly leaked into the extracellular fluid, which were determined through the degrees of contrast enhancement in the organs. This means that the 3 nm hydrophilized iron oxide nanoparticles enhanced vascular contrast for ∼1-2 h. In conclusion, the 3 nm hydrophilized iron oxide nanoparticles enable image observation for a prolonged time and are expected to be useful for a T1 contrast agent that targets lesions.

## Claims

1. A T1 contrast agent for magnetic resonance imaging comprising fine iron oxide nanoparticle cores and micelles encapsulating the core particles wherein the micelles comprise a nonionic surfactant consisting of a hydrophilic moiety containing at least two chains and a hydrophobic moiety containing at least one hydrocarbon chain.

2. The T1 contrast agent according to claim 1, wherein the nonionic surfactant is a compound represented by Formula 1:
[Formula 1] **A-O-C(O)-B**
wherein **A** is a hydrophilic moiety comprising two or more chains, each of which has one or more terminal -OH groups and comprises -O-(CH₂)ₙ- (wherein n is an integer from 1 to 10) or a C₃-C₅ alicyclic hydrocarbon group, the two or more of these being optionally bonded to each other to form a chain structure, and **B** is a hydrophobic moiety having a C₆-C₃₀ hydrocarbon chain structure.

3. The T1 contrast agent according to claim 1, wherein the cores have a diameter of 6 nm or less and a polydispersity index (PDI) of 0.2 or less.

4. The T1 contrast agent according to claim 1, wherein the hydrophilic moiety contains a polyalkylene glycol chain.

5. The T1 contrast agent according to claim 4, wherein the nonionic surfactant is a polysorbate surfactant.

6. The T1 contrast agent according to claim 1, wherein the contrast agent has an overall diameter of 10 nm or less and a PDI of 0.2 or less.

7. A platform for multiplex assays with a magnetic resonance imaging T1 contrast effect, comprising fine iron oxide nanoparticle cores and micelles encapsulating the core particles wherein a linker compound is introduced on the surface of the cores and the micelles comprise a nonionic surfactant consisting of a hydrophilic moiety containing at least two chains and a hydrophobic moiety containing at least one hydrocarbon chain.

8. The platform according to claim 7, wherein the linker is a click reaction-inducing compound.

9. The platform according to claim 7, wherein the linker is an amphiphilic compound containing a hydrophobic moiety at one end thereof and a hydrophilic moiety bound with a click reactive material at the other end thereof.

10. A method for producing nanoparticles for multiplex assays, comprising:
providing fine iron oxide nanoparticle cores; modifying the core particles with a click reaction-inducing compound; providing a solution of micelles comprising a nonionic surfactant consisting of a hydrophilic moiety containing at least two chains and a hydrophobic moiety containing at least one hydrocarbon chain; dispersing the modified core particles in the micelle solution to render the modified core particles hydrophilic via ligand encapsulation; and binding a functionalizing material containing a click reactive moiety to the surface of the core particles to modify the surface of the core particles.

11. The method according to claim 10, wherein the nonionic surfactant is used in 15- to 25-fold molar excess relative to the hydrophobic material on the surface of the cores for hydrophilization.

12. The method according to claim 10, wherein the functionalizing material is selected from the group consisting of disease targeting agents, chelating agents, fluorescent materials, and mixtures thereof.

13. The method according to claim 10, further comprising density gradient centrifugation.

14. A method for producing nanoparticles for multiplex assays, comprising: providing fine iron oxide nanoparticle cores; providing a solution of micelles in which a nonionic surfactant consisting of a hydrophilic moiety containing at least two chains and a hydrophobic moiety containing at least one hydrocarbon chain is mixed with an amphiphilic material containing a functionalizing group; and dispersing the core particles in the micelle solution such that hydrolyzation of the core particles via ligand encapsulation and surface modification of the core particles with the functionalizing material are performed simultaneously.

15. The method according to claim 14, further comprising density gradient centrifugation.
